# EUROPEAN PATENT APPLICATION

(11) **EP 4 726 652 A1**
(43) Date of publication of application: **15.04.2026**
(21) Application number: 25207163.4
(22) Date of filing: 07.10.2025
(51) Int. Cl.: G06T 7/13

(54) **IMAGE ANALYSIS**

(30) Priority: 10.10.2024 US 202463705766 P; 16.09.2025 US 202519329673
(71) Applicant: Siemens Healthineers International AG, 6312 Steinhausen (CH)
(72) Inventor: CZEIZLER, Elena, 00390 Helsinki (FI); GARG, Aarohi, 00100 Helsinki (FI); LAAKSONEN, Hannu, 02130 Espoo (FI)
(74) Representative: Mathisen & Macara LLP

(57) **Abstract**

A training corpus 201 comprising patient image information that includes contoured patient structures for a plurality of patients can be accessed, wherein the contoured patient structures were contoured at a particular radiation treatment facility, followed by training 202 at least one autosegmentation machine learning model using the training corpus to provide a trained autosegmentation machine learning model. Unsegmented patient image information for the plurality of patients can be input 203 into the trained autosegmentation machine learning model followed by outputting a corresponding baseline set of automatically contoured patient image information for the plurality of patients. The patient image information that includes contoured patient structures for the plurality of patients can then be compared 204 with the baseline set of automatically contoured patient image information for the plurality of patients to identify at least one of problematic contours or problematic labels.

## Description

### CROSS-REFERENCE TO RELATED APPLICATIONS

This application claims the benefit of U.S. Provisional Application No. 63/705,766 filed October 10, 2024, which is incorporated herein by reference in its entirety.

### TECHNICAL FIELD

These teachings relate generally to image analysis.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

As part of developing a radiation treatment plan, it is useful to identify with particularity both treatment targets (such as a tumor) and body portions to be avoided (often referred to as organs-at-risk). The identification of such patient volumes in patient imagery (such as computed tomography images) is typically referred to as contouring or segmentation.

Some planning techniques to develop an energy treatment plan suggest using deep learning approaches.

### SUMMARY

In one aspect, the present invention provides a method according to claim 1. Optional features are specified in the dependent claims. In another aspect, the present invention provides apparatus according to claim 14. In a further aspect, the present invention provides a non-transitory computer-readable medium as defined in claim 15.

### BRIEF DESCRIPTION OF DRAWINGS

Various needs are at least partially met through provision of the method, apparatus, and non-transitory computer-readable medium for image analysis described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a flow diagram as configured in accordance with various embodiments of these teachings;
FIG. 4 comprises a flow diagram as configured in accordance with various embodiments of these teachings;
FIG. 5 comprises a screenshot as configured in accordance with various embodiments of these teachings;
FIG. 6 comprises a screenshot as configured in accordance with various embodiments of these teachings;
FIG. 7 comprises a workflow as configured in accordance with various embodiments of these teachings;
FIG. 8 comprises a table as configured in accordance with various embodiments of these teachings;
FIG. 9 comprises a box plot as configured in accordance with various embodiments of these teachings; and
FIG. 10 comprises a workflow as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Data-based approaches, such as deep learning-based models (sometimes referred to herein as DL-based models), are being suggested for use for various energy treatment planning tasks such as radiation treatment planning tasks. Examples in such regards include three-dimensional (3D) dose prediction and field geometry setting.

The data needed to train these DL-based models often needs to include structure contours.

Structure contours in the context of radiation treatment planning are essentially outlines or delineations of various anatomical structures and regions of interest within a patient's body as visualized on medical imaging such as CT (computed tomography) scans or MRI (magnetic resonance imaging) scans. These contours can be important for planning the delivery of radiation therapy to treat cancerous tumors while minimizing the dose to surrounding healthy tissues. Segmentation can be helpful, for example, to: (1) delineate a treatment target (where, for example, a primary tumor and any involved lymph nodes or other areas at risk of containing microscopic disease are contoured to define a target volume that comprises the region that needs to receive a therapeutic dose of radiation); and (2) to delineate a so-called organ-at-risk (OAR) (such as, for example, the heart, lungs, spinal cord, kidneys, liver, and other organs that need to be spared from excessive radiation to the greatest extent possible) (or parts of an organ-at-risk such as a parotid stem) or other non-anatomical structures such as implants, pacemakers, avoidance structures, boluses, and so forth.

The process of creating structure contours is often referred to as contouring and is typically performed by a radiation oncologist, often with the assistance of dosimetrists and medical physicists. Contouring requires a deep understanding of anatomy, the behavior of different types of cancer, and the principles of radiation biology. Some software tools used in radiation oncology allow for semi-automated contouring using image segmentation algorithms, but the final contours generally require careful review and adjustment by the clinical team to ensure accuracy.

By one approach, one can look to train a DL-based model using existing historical structure contouring information. Unfortunately, when selecting a set of patients from a clinic's database for this purpose, the quality of the structure contours can vary greatly depending on the level of experience of the planners and the contouring protocols utilized. In some cases, the contours may not fully conform with one or more contouring guidelines, and this can create problems for automatic data processing pipelines, such as machine learning model training and inference. Furthermore, in this historical record, structures might not be properly contoured, or they might be only partially contoured.

The applicant has also determined that, when there are several planners contouring structures within a given clinic, there can also be unwanted discrepancies or variations in the contours generated by these different individuals. These kinds of variations may not be easily detectable and may require a lot of manual work to render usable for training purposes. (That said, detecting historical information where large variations appear between the planners may be useful at the clinical level since this could indicate the need for clinic-level training and/or supervision regarding contouring practices.)

Generally speaking, these various embodiments can provide an automatic approach that checks the quality and consistency of structures' contours by using a pretrained autosegmentation model. This allows problematic contours to be identified for both standard treatment planning workflows and automatic treatment pipelines. These teachings can serve to streamline the data integration process and enhance the quality of treatment planning. Moreover, since the autosegmentation model can be trained to follow certain contouring protocols, these teachings can also automatically identify manual contours that deviate from those protocols.

The applicant has also determined that large variations can occur as regards the labels used for the same structures. For example, left parotid contours may have labels such as Left Parotid, parotid_l, parotid_L, L parotid, parotid left, parotidGld_l, and so forth. While none of those examples are per se incorrect, having a record replete with varying labels for identical structures can lead to confusion, misuse, and/or an inability to properly use existing historical information. In addition, labels may appear in different languages. These teachings can also support automatically curating labels to ensure consistent labeling.

By one approach, these teachings can provide for training and employing an auto segmentation machine learning model. The foregoing may comprise providing a training corpus comprising patient image information that includes contoured patient structures for a plurality of patients wherein, by one approach, (some, at least a substantial majority, or even all of) these contoured patient structures were contoured at a particular radiation treatment facility, and then training at least one autosegmentation machine learning model using that training corpus to provide a trained autosegmentation machine learning model. The foregoing training may comprise, for example, using at least one of a backward-error-propagation algorithm or a gradient descent optimization algorithm.

By one approach, these teachings can then provide for inputting unsegmented patient image information for the plurality of patients into that trained autosegmentation machine learning model and outputting a corresponding baseline set of automatically contoured patient image information for the plurality of patients. With the latter information now available, these teachings can then provide for comparing the patient image information that includes the manually-contoured patient structures for the plurality of patients with the baseline set of automatically contoured patient image information for the plurality of patients to identify at least one of a problematic contour and/or a problematic label.

A contour or label can be "problematic" when that contour or label deviates in some substantial way from a more expected norm or convention by, for example, containing voxels that do not belong to the structure or by not containing all voxels that should actually be included for that structure. For example, a contour may deviate in a substantial way by being only a partial contour (as may occur, for example, with a long structure that is only partially drawn for the part that is "in-field," and hence at risk of being irradiated; an example is the spinal cord, where the planner might only be interested in the part that is at risk who will accordingly ignore the rest of the spinal cord) and a label may deviate in a substantial way by constituting an alpha numeric string that is different than a desired target string. As another example, when a structure is quite far from the target, the planner may be interested only in monitoring the level of dose delivered to the general region and thus he/she may not carefully delineate that structure and instead might just draw a generic shape instead that follows the general shape of the structure but which does not carefully distinguish between structure and non-structure voxels.

By one approach, if desired, the aforementioned comparing can comprise detecting incomplete contouring within the patient image information that includes the contoured patient structures for the plurality of patients.

By one approach, if desired, the aforementioned comparing can comprise generating similarity scores (where, if desired, information regarding the similarity scores can be grouped on an organ-by-organ basis).

The latter may comprise, for example, on a per patient basis, selecting matching pairs of patient image information that includes contoured patient structures and corresponding automatically contoured patient image information and then determining at least one similarity score for each of the matching pairs. By one approach, this can comprise determining at least two similarity scores for each of the matching pairs (for example, by determining a first similarity score for each of the matching pairs corresponding to at least one full patient contour and determining a second similarity score for each of the matching pairs corresponding to only a partial patient contour).

So configured, these teachings will then accommodate determining a difference between the first similarity score and the second similarity score to identify a problematic contour (or label) based at least in part on that difference. By one approach, these teachings will accommodate comparing similarity scores (such as the similarity scores themselves or the aforementioned similarity score difference values) with a corresponding predetermined threshold to identify problematic contours and/or labels.

By one approach, these teachings will accommodate displaying information regarding the similarity scores on a user interface. So configured, a user can then decide whether to take follow-on corrective action. These teachings will also support facilitating automated responses to the identification of problematic contours and/or labels. For example, by one approach, upon detecting a problematic label, these teachings can provide for automatically correcting the problematic label.

When there are several planners contouring structures within a given clinic, there may be unwanted discrepancies or variations in those contours. Such variations are not easily detectable and would typically require considerable manual attention and work to identify. Detecting structure contours where large variations appear amongst that group of planners can serve to inform an administrator of a need for clinic-level training and synchronization of contouring practices.

By one approach, these teachings can include a non-transitory computer-readable medium comprising instructions stored thereon for carrying out one or more of the actions, steps, and/or functions described herein, such as, but not limited to, training and employing an autosegmentation machine learning model, which instructions, when executed on a processor, perform the steps of accessing a training corpus comprising patient image information that includes contoured patient structures for a plurality of patients, wherein the contoured patient structures were contoured at a particular radiation treatment facility, training at least one autosegmentation machine learning model using the training corpus to provide a trained autosegmentation machine learning model, inputting unsegmented patient image information for the plurality of patients into the trained autosegmentation machine learning model and outputting a corresponding baseline set of automatically contoured patient image information for the plurality of patients, and comparing the patient image information that includes contoured patient structures for the plurality of patients with the baseline set of automatically contoured patient image information for the plurality of patients to identify at least one of problematic contours or problematic labels.

These teachings can provide an automatic approach for checking the quality and consistency of contours by using a pretrained autosegmentation model. These teachings can facilitate identifying problematic contours for both standard treatment planning workflows and automatic treatment pipelines. These teachings can streamline the data integration process and enhance the quality of treatment planning. Moreover, since the autosegmentation models can be trained to follow certain contouring protocols (such as those that pertain to a particular radiation treatment facility), these teachings can serve to automatically identify manual contours that deviate from those protocols.

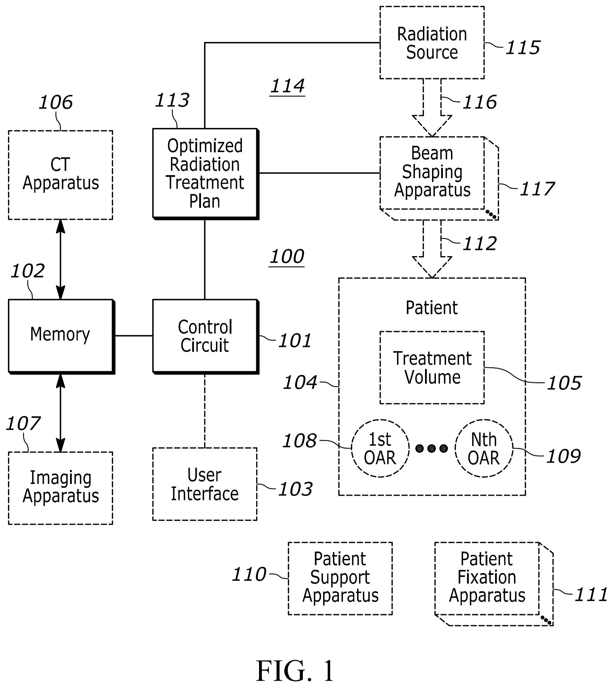

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another. In these regards, such multiple circuits may or may not be in close physical proximity to one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).) In particular, the control circuit 101 may be configured as an autosegmentation machine learning model as described herein.

In this illustrative example the control circuit 101 also operably couples to a user interface 103 (or to many user interfaces). This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 may be configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

These teachings can provide for an automatic approach for contouring quality assurance (QA) using one or more pretrained autosegmentation models, in which the models' predictions provide a set of baseline contours. These teachings can also provide for helping to ensure consistency as regards the labeling of contoured structures.

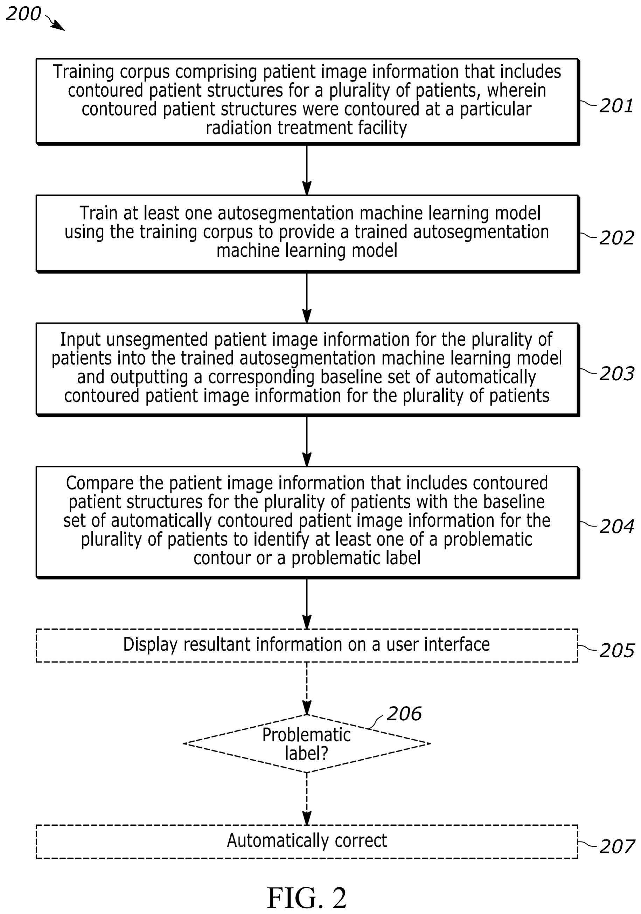

Referring now to FIG. 2, a process 200 that can be carried out (in whole or in part by the aforementioned control circuit 101 ) will be described.

At block 201, this process 200 provides a training corpus for a machine learning model. This training corpus comprises patient image information that includes contoured patient structures (such as one or more contoured target volumes and/or one or more contoured organs-at-risk) for a plurality of patients (i.e., different patients). By one approach, at least some of these patient structures were contoured at a particular radiation treatment facility. If desired, all of the contoured patient structures in the training corpus were contoured at that particular radiation treatment facility. By one approach, it may be beneficial for at least a substantial majority of those contoured patient structures to have been contoured without use of automated segmentation (in other words, those patient structures were fully contoured by a human).

At block 202, this process 200 provides for training at least one auto segmentation machine learning model using that training corpus to provide a trained autosegmentation machine learning model. As noted above, the aforementioned control circuit 101 may be configured, at least in part, to function as that trained autosegmentation machine learning model. By one approach, this training comprises using a backward-error-propagation algorithm. By another approach, in lieu of the foregoing or in combination therewith, this training comprises a gradient descent optimization algorithm.

At block 203, this process 200 provides for inputting unsegmented patient image information for the plurality of patients into the trained auto segmentation machine learning model and then outputting a corresponding baseline set of automatically contoured patient image information for the plurality of patients.

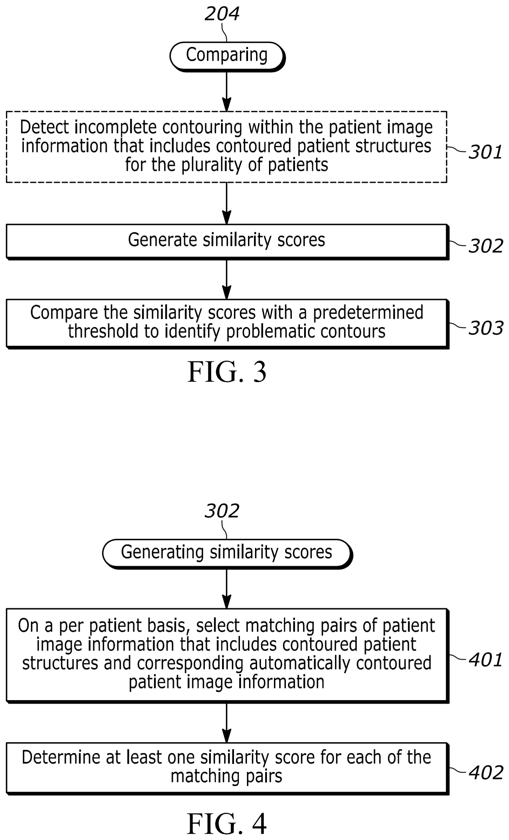

At block 204, this process then provides for comparing the patient image information that includes contoured patient structures for the plurality of patients with the baseline of automatically contoured patient image information for the plurality of patients to identify at least one of a problematic contour or a problematic label. Referring momentarily to FIG. 3, this comparison activity can optionally comprise, as illustrated at block 301, detecting incomplete contouring within the patient image information that includes contoured patient structures for the plurality of patients. An incomplete contour comprises a contour that fails to completely segment and thereby isolate a corresponding patient structure. By one approach, detection of incomplete contouring can be effected by way of the similarity score approach described below.

At block 302, this comparison process can provide for generating similarity scores. FIG. 4 provides some illustrative examples in these regards. At block 401, on a per patient basis, this activity can comprise selecting matching pairs of patient image information that includes contoured patient structures and corresponding automatically contoured patient image information and then, at block 402, determining at least one similarity score for each of the matching pairs.

By one approach, the latter comprises determining at least two similarity scores for each of the aforementioned matching pairs. This may comprise, for example, determining a first similarity score for each of the matching pairs that correspond to at least one full patient structure, and determining a second similarity score for each of the matching pairs that correspond to only a partial patient structure.

Referring again to FIG. 3, at block 303 the comparing activity can include comparing similarity scores with a predetermined threshold to identify problematic contours. By one approach, this can comprise determining a difference between the aforementioned first similarity score and the aforementioned second similarity score to identify a problematic contour based at least in part on that difference (for example, by comparing that difference with the aforementioned predetermined threshold).

So configured, these teachings will support using a trained autosegmentation model to automatically identify a pair of best matching contours from a collection of manually generated contours and corresponding automatically generated contours. Using this automatic pairing, these teachings will then further support generating a corresponding list of paired labels for the structures that correspond to those contours. This list can then be used to automatically curate/correct the manual labels, with or without first checking and approving those corrections by a human before any automatic correction is done. By one approach, a human can a be provided with the opportunity and capability of, for example, rejecting a proposed correction and/or editing a proposed correction.

By one approach, these teachings will accommodate comparing the patient image information that includes contoured patients structures for the plurality of patients with the baseline set of automatically contoured patient image information for the plurality of patients to identify problematic contours by generating similarity scores and grouping information regarding the similarity scores (such as differences therebetween) on an organ-by-organ basis.

Referring again to FIG. 2, by one optional approach and as illustrated at block 205, this process 200 will accommodate displaying resultant information corresponding to the identification of problematic contours and/or problematic labels on a user interface 103. Such visualization can serve, for example, to identify when, for a given structure, there are large variations within the evident contouring practices used at a given clinic. The latter may be evidenced by, for example, when the distribution of the similarity scores for a particular structure is very broad. In such a case, the administration for such a clinic may follow-up by revising contouring practices to ensure greater consistency. Visualization of such information can also serve to help identify when a particular structure is very systematically contoured (as evidenced, for example, by when the distribution of the similarity scores for that structure is very narrow).

If desired, these teachings will also accommodate, upon detecting a problematic label at block 206, automatically correcting the problematic label at block 207 to thereby ensure consistent labeling of contoured structures.

Further details that comport with these teachings will now be presented. It will be understood that the specific details of these examples are intended to serve an illustrative purpose and are not intended to suggest any particular limitations with respect to these teachings.

For the sake of illustrative examples, two use cases for checking the quality of patient organs-at-risk contours using a baseline set of contours provided by automated segmentation are: 1) flagging problematic contours for individual patients in the initial dataset, and 2) assessing the consistency of the contouring practices across the whole initial patient dataset. Further illustrative details in these regards are as follows. It will be understood that no particular limitations with respect to these teachings are intended by way of the specific details of these examples.

Use case 1: Using the set of baseline contours (automatically generated by one or more autosegmentation models) for flagging problematic contours in a selected patient dataset.

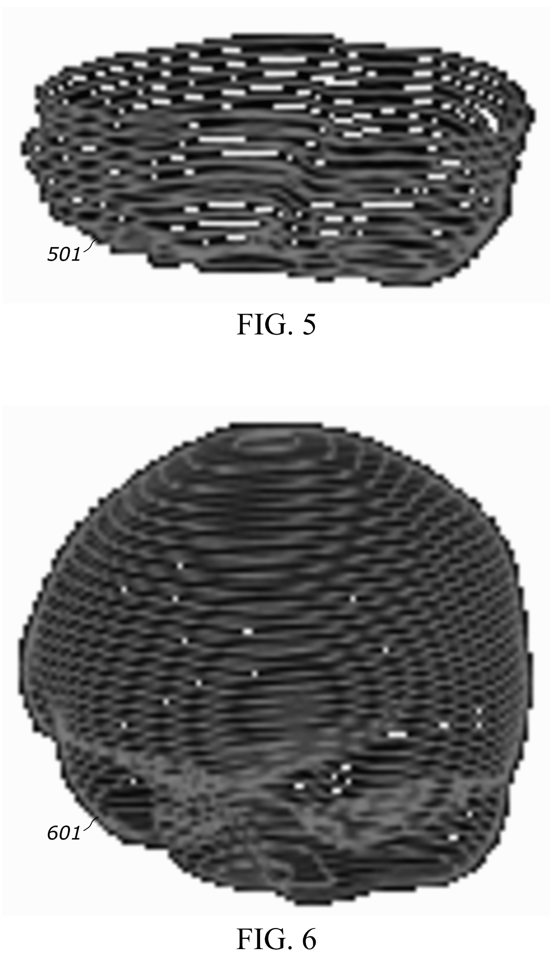

Illustrative problems one can detect with the present teachings include, for example, incomplete contouring of a given structure. By way of an illustrative example, FIG. 5 depicts a manually contoured brain that constitutes only a partial contour 501 of that structure, while Fig. 6 constitutes a complete contour 601 of that structure as generated by the autosegmentation algorithm. The present approaches can also permit detection of discrepancies relative to contouring standards that correspond, for example, to a particular radiation treatment facility.

In this first use case, and by one approach, automatically generated contours can be compared to corresponding manually generated contours using metrics such as DICE coefficients (which are a measure of the similarity between two sets, with the coefficient usually ranging from 0 to 1), the Hausdorff distance (which is a metric used in medical image segmentation that calculates the maximum distance between corresponding points on the boundary of patches belonging to the same class), and/or a mean surface distance. The following description will refer only to DICE coefficients for the sake of simplicity, but the same analysis can be done using other metrics including those mentioned above.

When the similarity score is within a predetermined range of a predefined threshold, the corresponding manually generated contours can be accepted by the system as being consistent with the contouring guidelines used by the autosegmenattion model. If the match does not correlate properly with the aforementioned threshold, the system can flag these instances for further review. By one approach, such a flag can initiate a user-in-the-loop process and the user will have the opportunity to correct the problem or ignore the structure as appropriate.

By one approach, in order to identify partial structures, the system can calculate the DICE coefficient in either of two different ways: by using the full structure or using only the common slices, i.e., the slices where the manually and automatically generated structures overlap. The system can then provide as an output a table with the two DICE scores mentioned above along with their difference. The system can have a predefined threshold for the computed DICE score difference (in particular, the difference between the two aforementioned DICE scores) that would indicate a structure that is only partially contoured as versus a fully contoured structure.

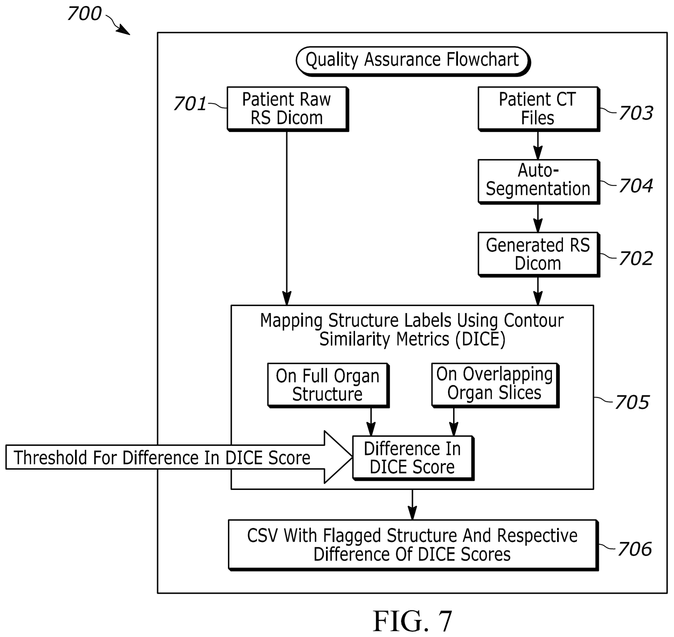

FIG. 7 presents a brief overview of a workflow 700 to effect quality assurance of structure contours by identifying incomplete contours.

This illustrative example presumes use of the manually partially contoured brain shown in FIG. 5 and the automatically fully countered brain shown in FIG. 6.

The former is available as patient raw RS DICOM information. (DICOM stands for Digital Imaging and Communications in Medicine, which is a global standard for storing, transmitting, and managing medical imaging data. In radiation oncology, a specialized subset called DICOM RT (Radiotherapy) was developed to handle the unique requirements of cancer treatment planning and delivery. In this context, "RS" refers to RT Structure Set, one of the core DICOM RT objects. The RT Structure Set defines the anatomical structures and regions of interest such as tumors, organs-at-risk, and reference points to be used in treatment planning.)

The latter (i.e., the automatically fully countered brain shown in FIG. 6) is available as generated RS DICOM information 702 (generated in this example by passing patient computed tomography files 703 through an auto segmentation process 704).

During processing 705, this process 700 determines that the manually generated contour 501 has a DICE score of 0.22 when compared to the fully contoured brain 601 that was automatically generated, whereas it has a DICE score of 0.98 when only the common slices are compared, indicating that the contour included in the patient planning files might be only partial.

This workflow 700 can then output a comma separated values file 706 containing information regarding flagged structures and respective differences of DICE scores, along with such other information as may be desired.

By one approach, and for visual comparison, the system could provide a review workspace (for example, via the above-described user interface 103) where an original and the automatically generated structures could be presented at the same time. If desired, overlapping volumes and non-overlapping volumes could be color coded to facilitate quick comparison by the user.

In the case when no contour of adequate quality is found for a particular structure, by one approach these teachings will accommodate providing the user with an opportunity to insert the automatically generated one into the patient record. This approach can help to ensure that no automatic data handling process gets broken due to missing data and can increase the quality of the data in machine learning training/inference use cases.

Use case 2 addresses assessing the consistency of contouring practices across the whole initial patient dataset.

This use case can begin with a set of patients and their corresponding sets of manually-derived contours. Automatically generated contours can then be compared to these patient structure contours using standard metrics such as DICE coefficients, Hausdorff distance, mean surface distance, or the like. The following description will again refer only to DICE coefficients for the sake of simplicity, but the same analysis can be done using other metrics including those mentioned above.

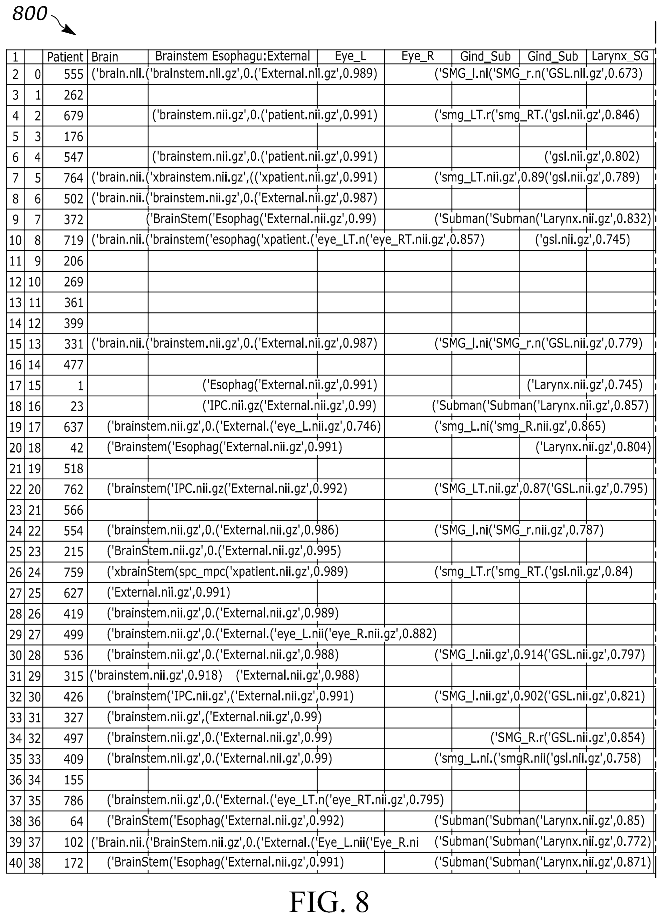

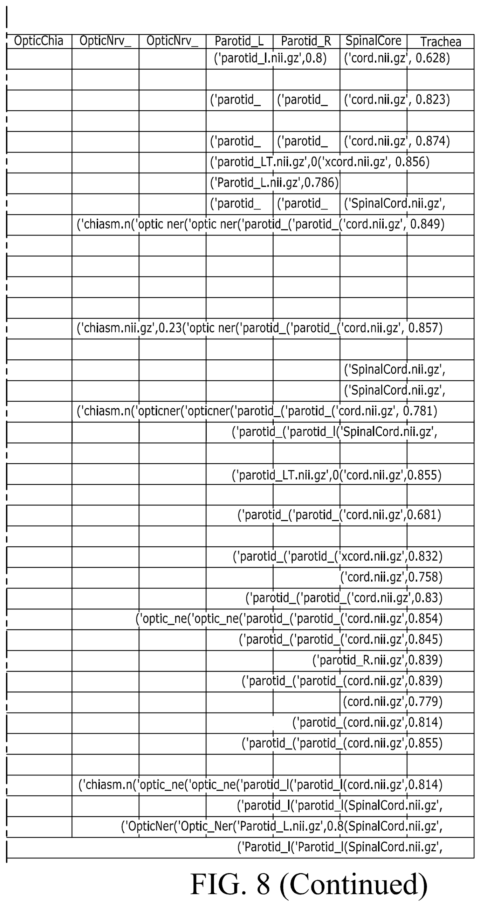

For each patient structure contour, these teachings can accommodate identifying the automatically generated contours having the highest DICE score. When these highest matching scores exceed predetermined organ-at-risk-specific thresholds, those matchings and the corresponding contours can be stored. FIG. 8 presents an illustrative example in these regards, in the form of a table 800 that maps pairs of contours with highest dice scores that exceed the applicable structure specific thresholds.

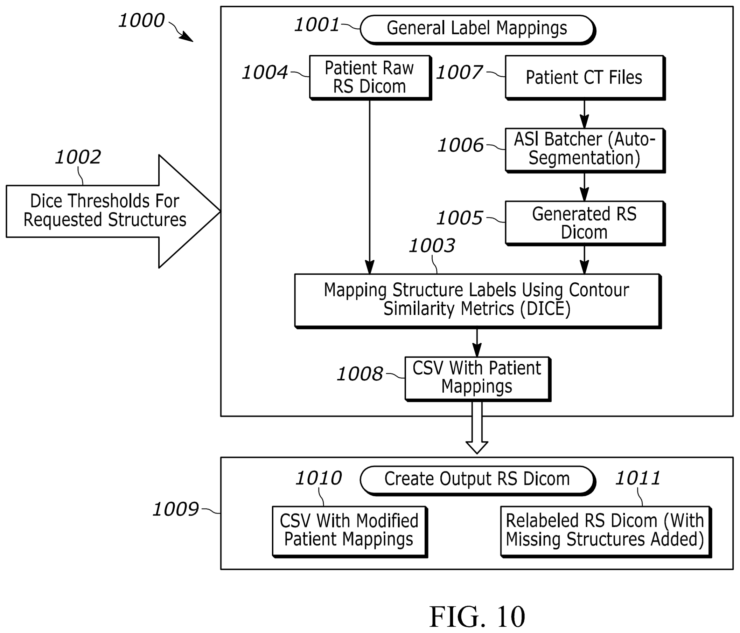

A further use case addresses an approach to label curation. Referring to FIG. 10, the illustrated workflow 1000 provides an example approach for identifying a pair of structures with the best DICE scores above a certain threshold. More particularly, this workflow 1000 generates label mappings at block 1001 using dice thresholds 1002 for requested structures. A structure labels mapping step 1003 receives patient raw RS DICOM information 1004 along with generated RS DICOM information 1005 provided by a pre-trained autosegmentation library (ASL) batcher 1006 that automatically segments structures from patient computed tomography files 1007. The resultant patient mappings 1008, provided, for example, in comma separated value (CSV) format, can then be used to create output RS DICOM information 1009. The latter can comprise, for example, CSV information with modified patient mappings 1010 as well as relabeled (that is, corrected) RS DICOM content with missing structures added thereto.

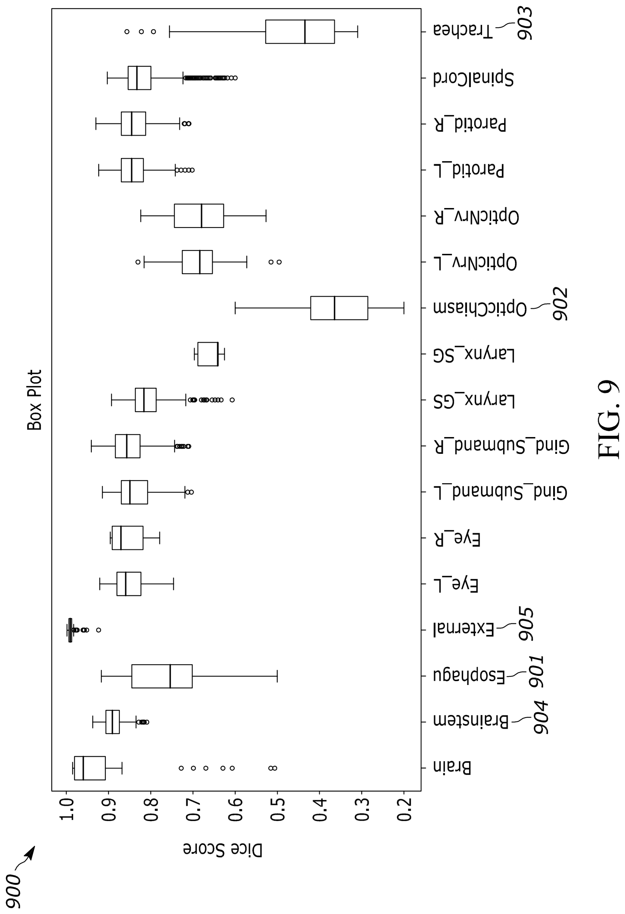

Once these computations are done (for example, for all patients and across all structures if desired), these teachings will then accommodate investigating the distributions of the computed values for each individual column in the aforementioned table 800. As an illustrative example in such regards, FIG. 9 presents a boxplot distribution 900 of the highest DICE scores for each organ. For structures such as the esophagus 901, the optic chiasm 902 (which is the part of the brain where the optic nerves cross), and the trachea 903, the range of the boxplot is more than 0.4, indicating a large variation in the contours for these structures across the whole dataset. That range implies some form of inconsistency in the contouring practices for that structure. In cases of the esophagus and the trachea, which are tubular structures, the discrepancy might be due to a different start and end point for the contouring slices whereas in structures such as the optic chiasm, the discrepancy might indicate a variation in the contours themselves. By contrast, the distributions for the brainstem 904 and external 905 are relatively narrow. That narrowness could indicate a higher level of consistency between the contours generated by the planners for these structures.

So configured, these teachings provide automatic approaches that can be used in clinics to help with a contouring quality assurance process. In particular, these teachings outline a machine learning-based approach that can assist the clinical staff with the contour review task and identify inconsistent contours.

Further aspects of these teachings are provided by the subject matter of the following clauses (where it will be understood that any of these clauses can be combined with any one of more of the other clauses as appropriate).

Clause 1. A method for training and employing an autosegmentation machine learning model, the method comprising: providing a training corpus comprising patient image information that includes contoured patient structures for a plurality of patients, wherein the contoured patient structures were contoured at a particular radiation treatment facility; training at least one autosegmentation machine learning model using the training corpus to provide a trained autosegmentation machine learning model; inputting unsegmented patient image information for the plurality of patients into the trained autosegmentation machine learning model and outputting a corresponding baseline set of automatically contoured patient image information for the plurality of patients; comparing the patient image information that includes contoured patient structures for the plurality of patients with the baseline set of automatically contoured patient image information for the plurality of patients to identify at least one of a problematic contour or a problematic label.

Clause 2. The method of clause 1 wherein training the at least one autosegmentation machine learning model comprises using at least one of: a backward-error-propagation algorithm; and a gradient descent optimization algorithm.

Clause 3. The method of clause 1 further comprising: upon detecting a problematic label, automatically correcting the problematic label.

Clause 4. The method of clause 1 wherein all of the contoured patient structures in the training corpus were contoured at the particular radiation treatment facility.

Clause 5. The method of clause 4 wherein at least a substantial majority of the contoured patient structures were contoured without automated segmentation.

Clause 6. The method of clause 1 wherein comparing the patient image information that includes contoured patient structures for the plurality of patients with the baseline set of automatically contoured patient image information for the plurality of patients to identify problematic contours comprises detecting incomplete contouring within the patient image information that includes contoured patient structures for the plurality of patients.

Clause 7. The method of clause 1 wherein comparing the patient image information that includes contoured patient structures for the plurality of patients with the baseline set of automatically contoured patient image information for the plurality of patients to identify problematic contours comprises: generating similarity scores: comparing the similarity scores with a predetermined threshold to identify the problematic contours.

Clause 8. The method of clause 1 wherein comparing the patient image information that includes contoured patient structures for the plurality of patients with the baseline set of automatically contoured patient image information for the plurality of patients to identify problematic contours comprises: generating similarity scores; grouping information regarding the similarity scores on an organ-by-organ basis.

Clause 9. The method of clause 8 further comprising: displaying information regarding the similarity scores on a user interface.

Clause 10. An apparatus for employing an autosegmentation machine learning model, the apparatus comprising: a control circuit configured to: input unsegmented patient image information for a plurality of patients into a trained autosegmentation machine learning model, wherein the trained autosegmentation machine learning model has been trained with a training corpus that comprises patient image information that includes contoured patient structures for a plurality of patients, wherein the contoured patient structures were contoured at a particular radiation treatment facility, and outputting a corresponding baseline set of automatically contoured patient image information for the plurality of patients; compare the patient image information that includes contoured patient structures for the plurality of patients with the baseline set of automatically contoured patient image information for the plurality of patients to identify at least one of problematic contours and problematic labels.

Clause 11. The apparatus of clause 10 wherein the control circuit is configured to compare the patient image information that includes contoured patient structures for the plurality of patients with the baseline set of automatically contoured patient image information for the plurality of patients to identify problematic contours by detecting incomplete contouring within the patient image information that includes contoured patient structures for the plurality of patients.

Clause 12. The apparatus of clause 10 wherein the control circuit is configured to compare the patient image information that includes contoured patient structures for the plurality of patients with the baseline set of automatically contoured patient image information for the plurality of patients to identify problematic contours by: generating similarity scores; comparing the similarity scores with a predetermined threshold to identify the problematic contours.

Clause 13. The apparatus of the clause 12 wherein the control circuit is configured to generate similarity scores by: on a per patient basis, selecting matching pairs of patient image information that includes contoured patient structures and corresponding automatically contoured patient image information; determining at least one similarity score for each of the matching pairs.

Clause 14. The apparatus of clause 13 wherein the control circuit is configured to determine at least two similarity scores for each of the matching pairs.

Clause 15. The apparatus of clause 14 wherein the control circuit is configured to determine the at least two similarity scores for each of the matching pairs by: determining a first similarity score for each of the matching pairs corresponding to at least one full patient structure; determining a second similarity score for each of the matching pairs corresponding to only a partial patient structure.

Clause 16. The apparatus of clause 15 wherein the control circuit is configured to determine a difference between the first similarity score and the second similarity score and to identify a problematic contour based at least in part on that difference.

Clause 17. The apparatus of clause 10 wherein the control circuit is configured to compare the patient image information that includes contoured patient structures for the plurality of patients with the baseline set of automatically contoured patient image information for the plurality of patients to identify problematic contours by: generating similarity scores; grouping information regarding the similarity scores on an organ-by-organ basis.

Clause 18. The apparatus of clause 17 wherein the control circuit is further configured to: display information regarding the similarity scores on a user interface.

Clause 19. A non-transitory computer-readable medium comprising instructions stored thereon for training and employing an autosegmentation machine learning model, which instructions, when executed on a processor, perform the steps of: accessing a training corpus comprising patient image information that includes contoured patient structures for a plurality of patients, wherein the contoured patient structures were contoured at a particular radiation treatment facility; training at least one autosegmentation machine learning model using the training corpus to provide a trained autosegmentation machine learning model; inputting unsegmented patient image information for the plurality of patients into the trained autosegmentation machine learning model and outputting a corresponding baseline set of automatically contoured patient image information for the plurality of patients; comparing the patient image information that includes contoured patient structures for the plurality of patients with the baseline set of automatically contoured patient image information for the plurality of patients to identify at least one of problematic contours or problematic labels.

Clause 20. The non-transitory computer-readable medium of clause 19 wherein at least a substantial majority of the contoured patient structures were contoured without automated segmentation.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A method for training and employing an autosegmentation machine learning model, the method comprising:
providing a training corpus comprising patient image information that includes contoured patient structures for a plurality of patients, wherein the contoured patient structures were contoured at a particular radiation treatment facility;
training at least one autosegmentation machine learning model using the training corpus to provide a trained autosegmentation machine learning model;
inputting unsegmented patient image information for the plurality of patients into the trained autosegmentation machine learning model and outputting a corresponding baseline set of automatically contoured patient image information for the plurality of patients;
comparing the patient image information that includes contoured patient structures for the plurality of patients with the baseline set of automatically contoured patient image information for the plurality of patients to identify at least one of a problematic contour or a problematic label.

2. The method of claim 1 wherein training the at least one autosegmentation machine learning model comprises using at least one of:
a backward-error-propagation algorithm; and
a gradient descent optimization algorithm.

3. The method of claim 1 or 2 further comprising:
upon detecting a problematic label, automatically correcting the problematic label.

4. The method of claim 1, 2 or 3 wherein all of the contoured patient structures in the training corpus were contoured at the particular radiation treatment facility.

5. The method of claim any one of claims 1 to 4 wherein at least a substantial majority of the contoured patient structures were contoured without automated segmentation.

6. The method of any one of claims 1 to 5 wherein comparing the patient image information that includes contoured patient structures for the plurality of patients with the baseline set of automatically contoured patient image information for the plurality of patients to identify problematic contours comprises detecting incomplete contouring within the patient image information that includes contoured patient structures for the plurality of patients.

7. The method of any one of claims 1 to 6 wherein comparing the patient image information that includes contoured patient structures for the plurality of patients with the baseline set of automatically contoured patient image information for the plurality of patients to identify problematic contours comprises:
generating similarity scores;
comparing the similarity scores with a predetermined threshold to identify the problematic contours.

8. The method of claim 7 including generating similarity scores by:
on a per patient basis, selecting matching pairs of patient image information that includes contoured patient structures and corresponding automatically contoured patient image information;
determining at least one similarity score for each of the matching pairs.

9. The method of claim 8 including determining at least two similarity scores for each of the matching pairs.

10. The method of claim 9 including determining the at least two similarity scores for each of the matching pairs by:
determining a first similarity score for each of the matching pairs corresponding to at least one full patient structure;
determining a second similarity score for each of the matching pairs corresponding to only a partial patient structure.

11. The method of claim 10 including determining a difference between the first similarity score and the second similarity score and to identify a problematic contour based at least in part on that difference.

12. The method of any one of claims 1 to 11wherein comparing the patient image information that includes contoured patient structures for the plurality of patients with the baseline set of automatically contoured patient image information for the plurality of patients to identify problematic contours comprises:
generating similarity scores;
grouping information regarding the similarity scores on an organ-by-organ basis.

13. The method of any one of claims 7 to 12 further comprising:
displaying information regarding the similarity scores on a user interface.

14. An apparatus for employing an autosegmentation machine learning model, the apparatus comprising:
a control circuit configured to:
input unsegmented patient image information for a plurality of patients into a trained autosegmentation machine learning model, wherein the trained autosegmentation machine learning model has been trained with a training corpus that comprises patient image information that includes contoured patient structures for a plurality of patients, wherein the contoured patient structures were contoured at a particular radiation treatment facility, and outputting a corresponding baseline set of automatically contoured patient image information for the plurality of patients;
compare the patient image information that includes contoured patient structures for the plurality of patients with the baseline set of automatically contoured patient image information for the plurality of patients to identify at least one of problematic contours and problematic labels; and,
optionally, wherein the control circuit is configured to perform the method of any one of claims 1 to 13.

15. A non-transitory computer-readable medium comprising instructions stored thereon for training and employing an autosegmentation machine learning model, which instructions, when executed on a processor, perform the steps of:
accessing a training corpus comprising patient image information that includes contoured patient structures for a plurality of patients, wherein the contoured patient structures were contoured at a particular radiation treatment facility;
training at least one autosegmentation machine learning model using the training corpus to provide a trained autosegmentation machine learning model;
inputting unsegmented patient image information for the plurality of patients into the trained autosegmentation machine learning model and outputting a corresponding baseline set of automatically contoured patient image information for the plurality of patients;
comparing the patient image information that includes contoured patient structures for the plurality of patients with the baseline set of automatically contoured patient image information for the plurality of patients to identify at least one of problematic contours or problematic labels; and,
optionally, wherein the instructions, when executed on the processor, perform the method of any one of claims 1 to 13.
